# EUROPEAN PATENT APPLICATION

(11) **EP 3 531 159 A1**
(43) Date of publication of application: **28.08.2019**
(21) Application number: 18158023.4
(22) Date of filing: 22.02.2018
(51) Int. Cl.: G01R 33/48, A61B 5/055, G01R 33/563

(54) **PATIENT-SPECIFIC PATTERN MODEL FOR CONTROL OF THE MR GUIDED RADIATION THERAPY**

(71) Applicant: Koninklijke Philips N.V., 5656 AE Eindhoven (NL)
(72) Inventor: UHLEMANN, Falk, 5656 AE Eindhoven (NL); NIELSEN, Tim, 5656 AE Eindhoven (NL); WUELBERN, Jan Hendrik, 5656 AE Eindhoven (NL); RAHMER, Jürgen Erwin, 5656 AE Eindhoven (NL)
(74) Representative: de Haan, Poul Erik

(57) **Abstract**

The present disclosure relates to a method for controlling a radiation therapy by a radiation therapy apparatus (200) of a target volume (246) in a subject (244) in accordance with an irradiation plan (199). The method comprises: generating a motion model of a movement of the subject (244), the motion model comprising patterns (326A-E) of values of at least one motion parameter (323); determining, during a predefined irradiation time window, current values of the motion parameter (323); determining if the distribution of the current values correspond to a given pattern of the patterns (326A-E) of the model; in response to determining that the distribution of the current values correspond to the given pattern and in case the given pattern is different from the pattern of the irradiation plan (199), modifying the irradiation plan in accordance with the given pattern, the modified irradiation plan becoming the irradiation plan.

## Description

### FIELD OF THE INVENTION

The invention relates to scanning imaging systems, in particular to a medical analysis system for controlling a radiation therapy by a radiation therapy apparatus of a target volume in a subject.

### BACKGROUND OF THE INVENTION

A magnetic resonance Linac (MR-Linac) system allows volumetric imaging of the patient during tumor treatment. This permits real-time monitoring of a target volume (e.g. a tumor) and patient or organ's movements thereby enabling the tracking of internal organs and target volume(s). Radiation treatment planning; however, is currently not performed in real-time during the treatment, but beforehand based on static or dynamic image data, e.g. one or multiple sets of 3D data. This approach assumes that pre-treatment data of a patient represents the intra-treatment situation. With respect to patient movement, this approach also assumes that the motion behavior stay constant. But it has been observed that different patients show different motion attitudes which change depending on that patient's state e.g. anxiety, tiredness.

### SUMMARY OF THE INVENTION

Even advanced, i.e. 4D data based, radiation plans do not take into account possible changes of the patient's motion (breathing) pattern over time. They are created before a radiotherapy course/session and cannot be easily updated during treatment. It is an insight of the inventors that therefore the validity of the model and thus the accuracy/efficiency of the treatment may decrease when the breathing pattern changes. It is an object of embodiments of the invention to improve radiation treatment, especially in cases where a tumor position is affected by breathing motion.

Embodiments of the invention relate to the generation of an improved motion model that describes a patient specific breathing motion. The motion model may be based on magnetic resonance imaging (MRI) data. These data may be acquired during the so-called simulation or pre-treatment phase on a diagnostic MRI system. Alternatively, during the simulation phase the MRI data for the motion model may be acquired by means of an MRI guided radiation therapy system. In cases where treatment is delivered by means of a MRI guided radiation therapy system, the MRI data acquired during the treatment may be used to update the motion model. The motion model may for example be created by the MRI system that acquired the MRI data, but also by a separate computer program product, e.g. on a separate work-station or in the cloud.

The motion model may be used for improvement of treatment guidance. Various embodiments provide for a method for controlling a radiation therapy by a radiation therapy apparatus, medical analysis system, and computer program product, as described by the subject matter of the independent claims. Advantageous embodiments are described in the dependent claims. The generation of the motion model and its use are the result of similar inventive concepts. However, most likely the motion model is generated by a different apparatus than the apparatus that will use the model for treatment guidance.

The present disclosure discloses a method to build an incrementally refined patient specific breathing motion model which can be used to control an MR radiation delivery by providing information about conformity of the current motion pattern with the therapy plan. It contains information about different motion patterns, e.g. their occurrence and transition probabilities and is improved iteratively during sessions to increase robustness and statistical information about the motion patterns. This information can be used for e.g. an irradiation stop, switching between different plans or for predicting following motion states (e.g. used for a dynamic multileaf collimator (MLC) control).

In one aspect, the invention relates to a medical analysis system for controlling a radiation therapy by a radiation therapy apparatus of a target volume in a subject. The system comprises a memory for storing machine executable instructions and a processor for controlling the system. The execution of the machine executable instructions causes the processor to: generate (e.g. prior to perform irradiation of the target volume) a motion model of a movement of the subject. The motion model comprises a plurality of patterns of values of at least one motion parameter (or of a set of or more motion parameters). Current values of the at least one motion parameter may be determined during irradiation of the target volume in accordance with an (initial) irradiation plan or therapy plan. The irradiation plan is defined using a predefined pattern of the at least one motion parameter. In case the distribution of the current values corresponds to a given pattern out of the plurality of patterns of the motion model, and in case the given pattern is different from the predefined pattern, the irradiation plan maybe modified in accordance with the given pattern.

In one example, modifying the irradiation plan comprises switching or replacing the irradiation plan by another irradiation plan. The other irradiation plan may be defined in accordance with the given pattern e.g. in this case the modified irradiation plan may be the other radiation plan.

The modified irradiation plan becomes the irradiation plan to be used for the irradiation of the target volume. If the comparison reveals that none of the patterns of the motion model corresponds to the current values of the motion parameters, the irradiation may be continued as before using the (initial) irradiation plan.

The motion of the subject such as a patient may be induced by different sources. One example source that induces the motion of the subject is the breathing by the subject. The motion of the subject in this case may be quantified by using breathing parameters as motion parameters for quantifying the motion of the subject.

The term "pattern" refers to the occurring or recurring of predefined values of the at least one motion parameter over a time interval. For example, the pattern may be defined by the time interval and the values of the at least one motion parameter over the time interval.

The at least one motion parameter may comprise a set of motion parameters. A subset of the motion parameters may be measured such as the breathing amplitude and another subset of motion parameters may be computed or derived, for example, from the measured parameters. For example, the breathing amplitude maybe measured or recorded over a recording time period and motion parameters (such as the harmonic frequency) that describe the variation of the amplitude over time may be derived from the measured amplitudes over a predefined time interval which is a subset of the recording time period. This may result in patterns over time intervals of the recording time period. A same pattern may occur a number of times during the recording time period. This may enable to define the occurrence probability of the pattern e.g. as the fraction of the number of occurrences of the total number of patterns determined over the recording time period. Each pattern of the motion model may be defined or associated with the values of the set of motion parameters that are defined for the time interval of the pattern.

The motion model may for example be created or generated during the radiation planning phase before the irradiation of the target volume starts. During irradiation of the target volume at least part of the set of motion parameters maybe evaluated during a time window in order to obtain current values of the at least part of the set of motion parameters over the time window. In order to determine if the distribution of the current values corresponds to one of the patterns of the motion model, the current values of the motion parameters are compared with values of corresponding motion parameters of each of the patterns of the motion model. For example, if the motion model is determined using the breathing amplitude and the fundamental frequency, during irradiation the current values of the amplitude and/or fundamental frequency may be determined over the time window, and the current values of the amplitude maybe compared with the amplitude values of each pattern of the motion model and the current values of the fundamental frequency may be compared with the fundamental frequency values of each pattern of the motion model. This may for example determine if the distribution of the current amplitudes corresponds to the amplitudes in a pattern of the motion model.

The comparison of two values of a motion parameter e.g. the current value (v1) compared with a value (vref) of the motion parameter of a given pattern of the motion model. If the difference |v1-vref| is smaller than a predefined delta, the two values v1 and vref may be considered similar values or corresponding values. A predefined delta may for example be user defined for each motion parameter. The current values are corresponding to a pattern of the motion model means that the difference between the current values and the values of the corresponding parameters of the pattern are smaller than the respective deltas.

By contrast to conventional methods (e.g. 4D data based), where radiation plans do not take into account possible changes of the patient's motion (breathing) over time, with the present disclosure, the patient's motion may be modeled such that the accuracy and efficiency of the treatment is at least maintained e.g. in particular when the breathing pattern changes in an unexpected manner.

According to one embodiment, each pattern of the patterns indicates or is provided in association with the values of the motion parameter over a respective time interval. The time intervals are consecutive time intervals. Each pattern may indicate over the respective time interval the one or more values of the motion parameter. For example, each pattern may indicate the distribution of the values of the motion parameter over the respective time interval. The time intervals of the patterns of the motion model may belong to the recoding time period and maybe consecutive within the recording time period. For example, the recording time period may be divided into the time intervals, wherein each time interval may be associated with a pattern. The time intervals may or may not be of the same duration. This embodiment may enable an accurate motion quantification as it does not leave gaps in the recording time period where no patterns are identified.

For example, a data structure such as data table may be provided, wherein each entry or record in the data structure corresponds to a pattern of the motion model and comprises the values of the set of motion parameters of the pattern and the respective time interval e.g. each record of the data structure may be associated with a pattern ID of the respective pattern.

According to one embodiment, each pattern of the patterns is associated with a probability of occurrence, wherein the given pattern is a pattern having the probability of occurrence higher than a predefined threshold. The comparison of the current values of the motion parameters with the motion model may be conditional in that only patterns having the occurrence probability higher than the predefined threshold are used for comparison with the current values of the motion parameters. This may prevent using rare patterns (e.g. fake patterns) which do not reflect the real movement of the subject.

According to one embodiment, the motion model comprises probability of transitions between successive patterns of the patterns. The execution of the instructions further causes the system to: determine a future pattern depending on the transition probabilities and the predefined pattern. In one example, the future pattern may be determined using previous patterns. The previous patterns may be advantageous because the transition probability to future pattern may by derived from the particular sequence of previous patterns and the previous transitions. The modification of the irradiation plan comprises modifying the irradiation plan in accordance with the future pattern. For example, the ongoing irradiation is based on the irradiation plan that is defined based in the predefined pattern. The predefined pattern maybe defined using values of the set of motion parameters as described above over a respective time interval. This may be performed by comparing the predefined pattern with the patterns of the motion model and identifying one pattern of the motion model that corresponds to the predefined model. The identified pattern maybe associated with a transition probability that indicates the probability that the identified pattern is going to transit to the future pattern. The comparison between the predefined pattern and the patterns of the motion model comprises comparing the values of the motion parameters of the predefined pattern with values of the corresponding parameters of each of the patterns of the motion model. The predefined pattern is considered as corresponding with a pattern of the model if the differences of the values of the motion parameters of the predefined pattern and values of the corresponding parameters are smaller than respective predefined deltas.

This embodiment may further increase the accuracy of the therapy being performed because the patterns to be used are determined beforehand and the change in the irradiation plan is anticipated.

According to one embodiment, the current values of the motion parameter are determined in a predefined time window, the time window being a sub interval of the total signal acquisition time interval of the given pattern. The total signal acquisition time interval is the time interval of the given pattern. Choosing the time window in accordance with the time interval of the given pattern (e.g. the time window is smaller than the time interval of the given patterns by a time period P1) may enable to use the optimal irradiation plan at least during the remaining time (P1) of the time interval of the given pattern that is not covered by the time window.

In one example, for determining the current values, the time window maybe chosen or varied and for each time window the current values are determined. The different time windows may or may not have the same starting time point. The end time point of the time window may for example be the current point of time of the irradiation. The time window may be varied such that it corresponds to a time interval of the time intervals of the patterns of the motion model.

According to one embodiment, the execution of the machine executable instructions further causes the processor to add a pattern to the patterns of the model having predefined unusual values of the at least one motion parameter, wherein in case the given pattern is the added pattern the modifying of the irradiation plan is performed such that the irradiation is stopped. This may enable an optimal control of the irradiation of the target volume.

According to one embodiment, the motion model is a model that is modeling the motion induced by the breathing of the subject, wherein the motion parameter comprises one of: the fundamental frequency, the harmonic frequency, other principal or independent frequency components in the signal spectrum, the breathing amplitude, drift or offset of the motion.

The set of motion parameters that are used to determine the motion model may be evaluated over time intervals as described above e.g. the drift may for example indicate the variation of the level of the baseline of the signal formed by the values of the breathing amplitude over a time interval.

According to one embodiment, the execution of the machine executable instructions further causes the processor to determine the values of the motion parameter using magnetic resonance image data acquired from the subject. For example, during the planning session or planning phase, patient motion is recorded over a sufficiently long (e.g. 10 min) duration which is the recording time period. This can be achieved by image based methods, e.g. navigator signals/images possibly interleaved/simultaneously with other imaging sequences, a breathing belt or optical systems. This means that planning scan and breathing monitoring can be performed in parallel without prolonging examination time. This may enable to seamlessly integrate this embodiment in MR guided radiation therapy apparatuses.

According to one embodiment, the execution of the machine executable instructions further causes the processor to update the motion model during the irradiation using measured values of the at least one motion parameter. For example, the measured values of the at least one motion parameter may be processed (as for generating the motion model) to determine one or more patterns that can be added to the motion model. This may enable an iterative improvement and adaptation to a patient specific motion. This may provide a multi-breathing-pattern model that may cover also radiotherapy sessions by recording patient movements and representing changes or irregularities of the motion pattern by occurrence and transition probabilities. This information can be employed for more accurate and reliable motion prediction, dynamic plan selection and (emergency) beam control based on real-time information during treatment.

According to one embodiment, the execution of the machine executable instructions further causes the processor to generate the motion model by: measuring values of the at least one motion parameter over time (over the recoding time period); generating clusters of the values using a predefined clustering method; providing the clusters as the patterns of the motion model. The at least one motion parameter comprises a number of motion parameters higher than a predefined minimum number (e.g. 1). This embodiment may particularly be advantageous in case of multiple motion parameters.

According to one embodiment, the clustering method comprises K-means. For example, k-centroids maybe determined by predefined values of the motion parameters. For example, at least part of the set of parameters described above may be used by the clustering method.

According to one embodiment, a magnetic resonance image-guided radiation therapy apparatus for controlling irradiation of a target volume is provided. The apparatus comprises a magnetic resonance imaging system and the medical analysis system of any of the preceding embodiments.

In another aspect, the invention relates to a method for controlling a radiation therapy by a radiation therapy apparatus of a target volume in a subject in accordance with an irradiation plan. The method comprises: generating a motion model of a movement of the subject, the motion model comprising patterns of values of at least one motion parameter; determining, during a predefined irradiation time window, current values of the motion parameter; determining if the distribution of the current values correspond to a given pattern of the patterns of the model; in response to determining that the distribution of the current values correspond to the given pattern and in case the given pattern is different from the pattern of the irradiation plan, modifying the irradiation plan in accordance with the given pattern, the modified irradiation plan becoming the irradiation plan.

In another aspect, the invention relates to a computer program product comprising machine executable instructions for execution by a processor, wherein execution of the machine executable instructions causes the processor to the methods of any of the preceding embodiments.

It is understood that one or more of the aforementioned embodiments of the invention may be combined as long as the combined embodiments are not mutually exclusive.

### BRIEF DESCRIPTION OF THE DRAWINGS

In the following preferred embodiments of the invention will be described, by way of example only, and with reference to the drawings in which:
Fig. 1 is a schematic diagram of a medical analysis system,
Fig. 2 shows a cross-sectional view of a magnetic resonance image-guided radiation therapy apparatus,
Fig. 3 is a flowchart of a method for controlling a radiation therapy by a radiation therapy apparatus of a target volume in a subject,
Fig. 4 is a flowchart of a method for building a motion model.

### DETAILED DESCRIPTION OF THE EMBODIMENTS

In the following, like numbered elements in the figures are either similar elements or perform an equivalent function. Elements which have been discussed previously will not necessarily be discussed in later figures if the function is equivalent.

Various structures, systems and devices are schematically depicted in the figures for purposes of explanation only and so as to not obscure the present invention with details that are well known to those skilled in the art. Nevertheless, the attached figures are included to describe and explain illustrative examples of the disclosed subject matter.

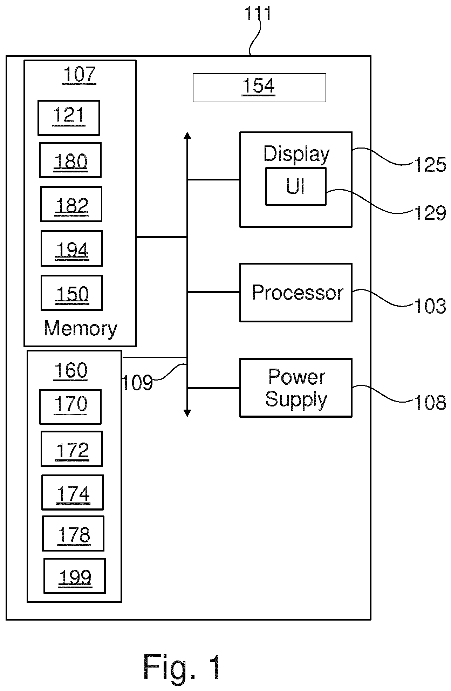

Fig. 1 is a schematic diagram of a medical analysis system 111. The medical analysis system 111 comprises a processor 103, a memory 107 each capable of communicating with one or more components of the system 111. For example, components of the medical analysis system 111 are coupled to a bidirectional system bus 109.

It will be appreciated that the methods described herein are at least partly non-interactive, and automated by way of computerized systems. For example, these methods can further be implemented in software 121, (including firmware), hardware, or a combination thereof. In exemplary embodiments, the methods described herein are implemented in software, as an executable program, and is executed by a special or general-purpose digital computer, such as a personal computer, workstation, minicomputer, or mainframe computer.

The processor 103 is a hardware device for executing software, particularly that stored in memory 107. The processor 103 can be any custom made or commercially available processor, a central processing unit (CPU), an auxiliary processor among several processors associated with the medical analysis system 111, a semiconductor based microprocessor (in the form of a microchip or chip set), a macroprocessor, or generally any device for executing software instructions. The processor 103 may control the operation of a magnetic resonance image-guided radiation therapy apparatus to which the medical analysis system 111 is connectable e.g. via a hardware interface 154.

The memory 107 can include any one or combination of volatile memory elements (e.g., random access memory (RAM, such as DRAM, SRAM, SDRAM, etc.)) and nonvolatile memory elements (e.g., ROM, erasable programmable read only memory (EPROM), electronically erasable programmable read only memory (EEPROM), programmable read only memory (PROM). Note that the memory 107 can have a distributed architecture, where various components are situated remote from one another, but can be accessed by the processor 103. Memory 107 may store an instruction or data related to at least one other constituent element of the system 111.

The medical analysis system 111 may further comprise a display device 125 which displays characters and images and the like e.g. on a user interface 129. The display device 125 maybe a touch screen display device.

The medical analysis system 111 may further comprise a power supply 108 for powering the medical analysis system 111. The power supply 108 may for example be a battery or an external source of power, such as electricity supplied by a standard AC outlet.

The medical analysis system 111 may be configured to connect to a magnetic resonance image-guided radiation therapy apparatus (not shown) comprising a scanning imaging system such as MRI, CT and PET-CT imagers and a radiotherapy module.

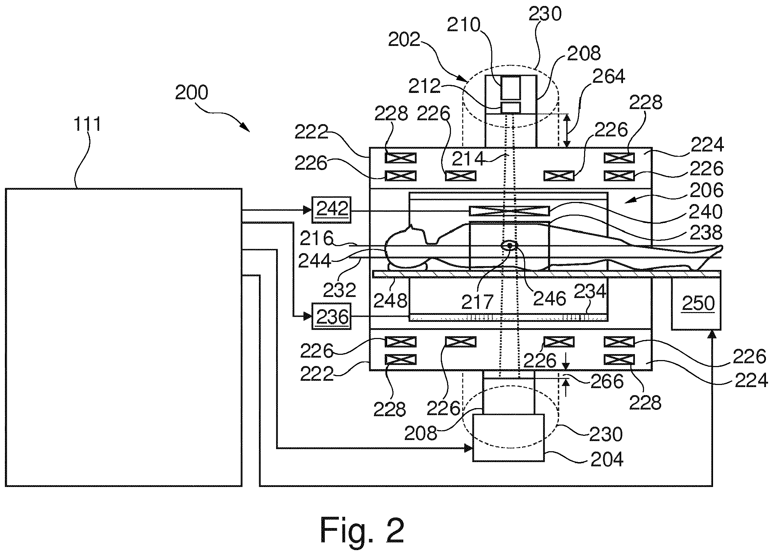

The connection between the medical analysis system 111 and the magnetic resonance image-guided radiation therapy apparatus may for example comprise a BUS Ethernet connection, WAN connection, Internet connection etc. The medical analysis system 111 and the magnetic resonance image-guided radiation therapy apparatus may or may not be an integral part. In other terms, the medical analysis system 111 may or may not be external to the magnetic resonance image-guided radiation therapy apparatus. The magnetic resonance image-guided radiation therapy apparatus comprises components that may be controlled by the processor 103 in order to configure the magnetic resonance image-guided radiation therapy apparatus. The configuration of the magnetic resonance image-guided radiation therapy apparatus may enable the operation of the magnetic resonance image-guided radiation therapy apparatus. The operation of the magnetic resonance image-guided radiation therapy apparatus may for example be automatic. Figure 2 shows an example of components of the magnetic resonance image-guided radiation therapy apparatus. In one example, the magnetic resonance image-guided radiation therapy apparatus may be configured to provide output data such as images in response to a specified measurement.

The processor 103 may be adapted to receive information from the magnetic resonance image-guided radiation therapy apparatus in a compatible digital form so that such information may be displayed on the display device 125. Such information may include operating parameters, alarm notifications, and other information related to the use, operation and function of the magnetic resonance image-guided radiation therapy apparatus.

Storage device 160 is shown as containing image magnetic resonance data 170 that have been acquired by the magnetic resonance imaging module. The storage device 160 is shown as further containing diagnostic images (i.e. image representation) 172 that have been reconstructed from the image magnetic resonance data. The storage device 160 is shown as further containing coordinates 174 of a target volume of a subject. The storage deice 160 is shown as further containing radiotherapy control signals 178.

The memory 107 is shown as containing a therapy system control module 180. The therapy system control module 180 contains machine executable instructions which allow the processor 103 to control the overall functioning of the magnetic resonance image-guided radiation therapy apparatus. The memory 107 is shown as further containing a radiotherapy apparatus control module 182. The radiotherapy apparatus control module 182 contains machine executable instructions which allow the processor 103 to control the functioning of the radiotherapy module.

The memory 107 is shown as further containing radiotherapy control signal generation module 194. The radiotherapy control signal generation module 194 contains computer executable code which the processor 103 uses to generate the radiotherapy control signals 178. The radiotherapy control signals 178 may be generated in conjunction with the coordinates 174 of the target volume.

The memory 107 may further comprise a motion compensation component 150 for performing at least part of the present method. The motion compensation component 150 may or may not be part of software component 121. The motion compensation component 150 may be configured to control at least part of the modules 180-194 for performing at least part of the present method.

The medical analysis system 111 may be configured to receive a treatment or irradiation plan 199 for irradiating the target volume, and may generate control signals e.g. using radiotherapy control signal generation module 194. The control signals may be used for controlling the radiotherapy module to irradiate the target volume using the control signals, in accordance with the location of the target volume.

Fig. 2 shows a cross-sectional view of a magnetic resonance image-guided radiation therapy apparatus 200. The apparatus 200 is shown as comprising a radiotherapy module 202 and a magnetic resonance imaging module 206. The radiotherapy module 202 comprises a ring mechanism 208. The ring mechanism 208 supports a radiotherapy source 210. The radiotherapy source 210 is representative and may be a LINAC x-ray source, an x-ray 2 and a radioisotope gamma radiation source. Adjacent to the radiotherapy source 210 is a multi-leaf beam collimator 212 for collimating a radiation beam 214 that is generated by the radiotherapy source 210. The ring mechanism 208 is also adapted for moving e.g. rotating the radiotherapy source 210 and the beam collimator 212 about a rotational point 217 of the radiotherapy module 202. A rotational axis 216 passes through the rotational point 217.

The magnetic resonance imaging module 206 is shown as comprising a main magnet 222. The ring mechanism 208 is ring-shaped and surrounds the main magnet 222. The main magnet 222 shown in Figure 2 is a cylindrical type superconducting magnet. However, other magnets are also applicable for embodiments of the invention. The main magnet 122 has a supercooled cryostat 224. Inside the cryostat 224 there is a collection of superconducting coils 226. There are also compensation coils 228 whose current opposes the direction of current in the superconducting coils 226. This creates a low magnetic field zone 230 that circles or encompasses the main magnet 222. The cylindrical main magnet 222 is shown as having an axis 232 of symmetry.

Within the bore of the magnet there is a magnetic field gradient coil 234 which is used for acquisition of image magnetic resonance data to spatially encode objects within an imaging volume 238 of the main magnet 222. The magnetic field gradient coil 234 is connected to a magnetic field gradient coil power supply 236. The magnetic field gradient coil 234 is intended to be representative. Typically magnetic field gradient coils contain three separate sets of coils for spatially encoding in three orthogonal spatial directions. The imaging volume 238 is located in the center of the main magnet 222.

Adjacent to the imaging volume 238 is a radio frequency (RF) coil 240 for manipulating the orientations of magnetic spins within the imaging volume 238 and for receiving radio transmissions from spins also within the imaging volume 238. The radio frequency coil 240 is connected to a radio frequency transceiver 242. The radio frequency coil 240 and radio frequency transceiver 242 may be replaced by separate transmit and receive coils and a separate transmitter and receiver. It is understood that the radio frequency coil 240 and the radio frequency transceiver 242 are simply representative.

Within the center of the main magnet 222 is also located a subject 244. The subject 244 has a target volume (or target zone) 246 and is shown as reposing on a patient carrier 248. The RF coil 240 may transmit RF pulses into the target volume 246. The patient carrier 248 has a mechanical positioning system 250. The mechanical positioning system 250 is adapted for positioning the patient carrier 248 within the main magnet 222. Depending upon the space available inside of the main magnet 222, the mechanical positioning system 250 may move the patient carrier 248 in different directions including a direction perpendicular to the magnet axis 232. If there is more space available inside the main magnet 222 the mechanical positioning system 250 may have more degrees of freedom. For instance the mechanical positioning system 250 may position the patient carrier 248 with six degrees of freedom.

The radio frequency transceiver 242, the magnetic field gradient coil power supply 236, the mechanical actuator 204, and the mechanical positioning system 250 are all shown as being connected to a hardware interface 254 of medical analysis system 111.

The radiotherapy module 202 may be, for example, connected to the hardware interface 154 and communicates with the medical analysis system 111 via the mechanical actuator 204 or via other means enabling communication between radiotherapy module 202 and the medical analysis system 111.

For the example shown in Figure 2, the rotational axis 216 of the radiotherapy module is not coaxial with the magnet axis 232. The rotational point 217 is shown as being off center from the magnet axis 232. It can be seen that the target zone 246 is off-center and away from the magnet axis 232. The radiotherapy apparatus 202 has been moved by mechanical actuator 204 such that the rotational point 217 of the radiotherapy module is within the target zone 246. It can be seen that the ring mechanism 208 has been moved relative to the magnet 222.

The radiation beam 214 passes through the rotational point 217. Placing the rotational point 217 at the center of the target zone 246 allows the target zone to be treated continuously when the radiation beam 214 is created by the radiotherapy source 210 and is rotated by the ring mechanism 208.

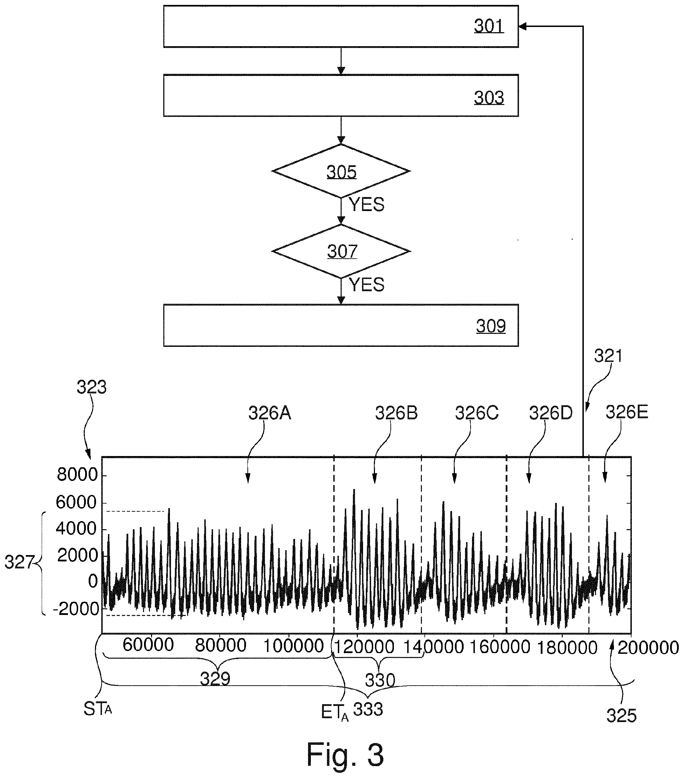

Fig. 3 is a flowchart of a method for controlling a radiation therapy by magnetic resonance image-guided radiation therapy apparatus 200 of the target volume 246.

In step 301, a motion model of a movement of the subject 244 may be generated. The motion model may be generated using a set of one or more motion parameters. The movement of the subject 244 may cause a change in the coordinates 174 of the target volume 246 and would thus be taken into account for irradiating the target volume 244. The motion model maybe specific to the subject 244.

The motion may for example be induced by the breathing of the subject 244. The set of motion parameters comprises at least one of the fundamental frequency, harmonic frequencies, other principal or independent frequency components in the signal spectrum (the signal spectrum may for example be defined as the Fourier transformed breathing amplitude signal), breathing amplitude, drift and offset of the motion. For example, multiple motion parameters maybe used to determine the motion model.

The motion model may be generated by recording data of the subject over a recording time period. The recorded data may for example comprise values of the set of motion parameters such as breathing amplitude 323 as shown in graph 321 of Figure 3. Graph 321 indicates the variation of the breathing amplitude 323 over time 325. The recording time period 333 is the time during which the breathing amplitude 323 of the subject 244 have been recorded or measured. The value of the breathing amplitude 323 over the recording time period may be used computer values of other motion parameters of the set of motion parameters.

The graph 321 indicates groups or clusters or patterns 326A, 326B, 326C, 326D and 326E of values of the breathing amplitude 323. For example, the pattern 326A comprises breathing amplitude values that vary within an amplitude range 327. The pattern 326A covers a time interval 329. The time interval 329 has a starting time ST_{A} point and an end time point ET_{A} (where A refers to the pattern 326A). The pattern 326B covers another time interval 330. The time interval 330 may or may not be the same as the time interval 329. Each pattern of the patterns 326A-E may be defined or described using the motion parameters such as the amplitude range 327, the start time ST and end time ET of the pattern, the harmonic frequencies or other parameters that may identify each pattern separately from other patterns.

The motion model may comprise the patterns 326A-E. For example, the motion model may comprise the motion parameter values (e.g. ST, ET and amplitude range) of each of the patterns 326A-E and an indication (e.g. pattern ID) of each pattern 326A-E.

Step 301 may be executed or performed offline while the target volume 246 is not irradiated. For example, during the planning session patient motion is recorded over a sufficiently long (e.g. 10 min) duration which is the duration of the recoding time period. This can be achieved by image based methods, e.g. navigator signals/images possibly interleaved/simultaneously with other imaging sequences, a breathing belt or optical systems. This means that planning scan and breathings monitoring can be performed in parallel without prolonging examination time.

For example, the graph 321 may be presented on the display device 125 to a user of the magnetic resonance image-guided radiation therapy apparatus 200. And in response to presenting the graph 321, the values of the motion parameters of the patterns 326A-D maybe received.

During irradiation of the target volume 246, current values of the set of motion parameters maybe determined in step 303. For example, the target volume 246 is irradiated in accordance with irradiation plan 199. The irradiation plan 199 may be defined based on a predefined pattern having respective values of the set of motion parameters. The predefined pattern may or may not be part of the patterns 326A-E of the motion model.

In inquiry step 305, it may be determined if the current values (e.g. distribution of current values) correspond to a given pattern of the patterns 326A-E of the motion model. For example, the current values of the breathing amplitude may be measured over a predefined time window. And at least part of the set of motion parameters e.g. the amplitude range 327, described above may be computed using the current values of the breathing amplitude 323. The current values of at least part of the set of parameters may be compared with the values of the corresponding parameters of the set of parameters of the patterns 326A-E of the motion model.

For example, the current value (v1) of the amplitude range 327 may be compared with the value (vref) of the amplitude range of each pattern of the motion model. And if the difference between v1 and vref of a given pattern 326A-E is smaller than a predefine range delta, the current value may be considered as corresponding to the value of the amplitude range of the given pattern. The current values are determined to correspond to a pattern of the motion model if the difference between the current values and corresponding values of the set of parameters of that pattern are all smaller than respective predefined deltas.

In response to determining that the distribution of or the current values correspond to the given pattern, it may be determined in inquiry step 307 if the given pattern is different from the predefined pattern used by the (ongoing) irradiation. This may be performed by comparing the values of the set of parameters of the given pattern with the values of the set of parameters of the predefined pattern.

In case the given pattern is different from the predefined pattern used for the irradiation, the irradiation plan 199 maybe modified in step 309 in accordance with the given pattern. The modified irradiation plan becomes then the irradiation plan to be used for the irradiation of the target volume 246. In another example, the irradiation plan 199 maybe replaced by another irradiation plan in step 309, wherein the other irradiation plan defined in accordance with given pattern.

The irradiation plan may be defined by evaluating predefined irradiation parameters. The irradiation parameters may for example comprise at least the coordinates of the target volume 246, the duration of the irradiation and the amount of the radiation to be used. For modifying the irradiation plan in step 309, a predefined plan that matches or that is associated with the breathing pattern maybe chosen as the (modified) irradiation plan.

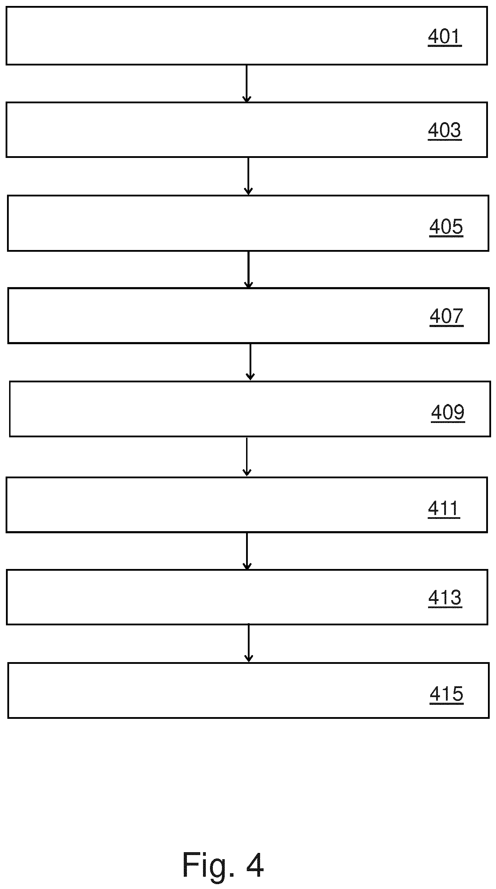

Fig. 4 is a flowchart of an example method for generating a motion model further detailing step 301.

In step 401, the fundamental frequency may be calculated in a sliding time window (over multiple breathing cycles) over the recorded data e.g. obtained during irradiation over the recording time period 333. The sliding time window may be varied depending on the encountered breathing pattern(s), and one or more passes over the recording time period might be performed. For example, in a first pass a first time window may be used and in a second pass a different second time window may be used. The need to use a second pass may for example be triggered by the need to determine further patterns or clusters. In step 403, the breathing amplitude maybe calculated in the sliding time window (over multiple breathing cycles) over the recorded data. In an optional step 405, the drift or offset of the breathing motion may be calculated (over multiple breathing cycles) over the recorded data. In step 407, the calculated parameters values of steps 401-405 maybe clustered using a clustering method such as, for example, K-means and thereby a feature/cluster space of clusters representing the recorded data maybe created. For example, the parameter values of step 401-405 as function of the respective time windows may be provided as input to the clustering method. In step 409, occurrence probabilities may be calculated for the clusters (or breathing patterns) during the recorded time period. In step 411, it may be created for each cluster of the clusters having an occurrence probability higher than a predefined threshold a therapy plan or irradiation plan for irradiating the target volume 246 in accordance with the respective cluster. In step 413, it may optionally added one or more clusters to represent motion patterns which may be "impossible", e.g. extremely high/low breathing frequencies or amplitudes which could be caused by errors during recording of the data being processed in steps 401-405. And any motion signal/pattern being classified into one of these "forbidden" clusters may result in a therapy halt. Based on the clusters and the calculated parameters values of steps 401-407, the transition probability between different patterns or clusters may be calculated in step 415.

After these steps 401-415 the motion model is created. The motion model comprises the patterns, the transition probabilities and the occurrence probabilities. In following treatment sessions, the motion model may be used to select the irradiation plan corresponding to the currently detected breathing pattern (e.g. characterized by frequency, amplitude and offset). In case the detected motion corresponds to a "forbidden" cluster, the therapy is paused or stopped and the user is notified. The data recorded during the sessions is also added to the initial motion data and a recalculation of the measures, clusters and probabilities can be performed. In case the new data indicates significant deviations, i.e. results in significantly different clusters and probabilities, a re-planning may be considered.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a 'circuit', 'module' or 'system'. Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

A 'computer memory' or 'memory' is an example of a computer-readable storage medium. A computer memory is any memory which is directly accessible to a processor. A 'computer storage' or 'storage' is a further example of a computer-readable storage medium. A computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the 'C' programming language or similar programming languages and compiled into machine executable instructions. In some instances, the computer executable code maybe in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the Internet using an Internet Service Provider).

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device'. A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 103: processor
- 107: memory
- 108: power supply
- 109: bus
- 111: control system
- 121: software
- 125: display
- 129: user interface
- 150: motion compensation module
- 170: image magnetic resonance data
- 172: diagnostic images
- 174: coordinates
- 178: radiotherapy control signals
- 180: therapy system control module
- 182: radiotherapy apparatus control module
- 194: radiotherapy control signal generation module
- 199: irradiation plan
- 200: MR guided therapy apparatus
- 202: radiotherapy module
- 204: mechanical actuator
- 206: magnetic resonance imaging module
- 208: ring mechanism
- 210: radio therapy source
- 212: multi-leaf beam collimator
- 214: radiation beam
- 216: rotational axis
- 217: rotational point
- 222: main magnet
- 224: cryostat
- 226: superconducting coil
- 228: compensation coil
- 230: low magnetic field zone
- 232: magnet axis
- 234: magnetic field gradient coil
- 236: magnetic field gradient coil power supply
- 238: imaging volume
- 240: radio frequency coil
- 242: radio frequency transceiver
- 244: subject
- 246: target volume
- 248: patient carrier
- 250: mechanical positioning system
- 301-309: method steps
- 321: graph
- 323: breathing amplitude
- 325: time
- 326: pattern
- 327: amplitude range
- 329-330: time interval
- 333: recording time period
- 401-415: method steps.

## Claims

1. A method for generating a motion model of a movement of a subject based on medical data describing the breathing motion of the subject, wherein the motion model comprises a plurality of potential breathing patterns of the subject, wherein the method comprises the following steps:
- dividing the medical data into multiple subsets and;
- calculating a fundamental frequency and / or breathing amplitude for each of the subsets and;
- classifying and / or clustering the subsets by means of their fundamental frequency and / or breathing amplitude, wherein the resulting clusters describe potential breathing patterns of the subject.

2. A method for generating a motion model according to claim 1, further comprising the step of:
- creating a radiotherapy plan based on the breathing patterns described by one or more of the clusters of subsets.

3. A method for generating a motion model according to claim 2, further comprising the step of:
- calculating occurrence probabilities for the clusters of subsets;
Wherein radiotherapy plans are only created for the clusters with the highest occurrence probability.

4. A method for generating a motion model according to any of the preceding claims, further comprising the step of:
- calculating an offset of the breathing motion for each of the subsets and use the offset as an additional parameter for classifying and / or clustering the subsets.

5. A method for generating a motion model according to any of the preceding claims, further comprising the step of:
- adding one or more clusters to the motion model, wherein the added clusters comprise a fundamental frequency, breathing amplitude and /or offset that did not occur in any of the subsets and
- tagging the one or more added clusters as an added cluster, such that during treatment delivery based on the motion model treatment can be stopped if a motion pattern is measured that corresponds to the one or more added cluster.

6. A method for generating a motion model according to any of the preceding claims, further comprising the step of:
- calculating a transition probability between the clusters, wherein the transition probability describes the probability that a subject changes its breathing pattern from a pattern described by a first cluster to a breathing pattern described by a second cluster.

7. A method for generating a motion model according to any of the preceding claims, further comprising the step of:
- acquiring the medical data describing the breathing motion.

8. A magnetic resonance imaging apparatus, comprising a memory (107) for storing machine executable instructions; and a processor for controlling the system, wherein execution of the machine executable instructions causes the processor to perform the method according to claim 7.

9. A computer program product comprising machine executable instructions for execution by a processor, wherein execution of the machine executable instructions causes the processor to perform the method of claim any of claims 1-7.

10. A magnetic resonance imaging guided radiation therapy system for controlling a radiation therapy by a radiation therapy apparatus (200) of a target volume (246) in a subject (244), the system (111) comprising a memory (107) for storing machine executable instructions; and a processor (103) for controlling the system (111), wherein execution of the machine executable instructions causes the processor (103) to:
- receive a motion model of a movement of the subject, wherein the motion model is based on medical data describing the breathing motion of the subject, wherein the motion model comprises a plurality of clusters describing the potential breathing patterns of the subject by their fundamental frequency and / or breathing amplitude and
- determine current values of the fundamental frequency and / or breathing amplitude (323) during irradiation of the target volume (246) in accordance with an irradiation plan (199), the irradiation plan (199) being defined using a predefined pattern of the fundamental frequency and / or breathing amplitude (323);
- determine if the distribution of the current values corresponds to a given pattern out of the plurality of patterns (326A-E) of the motion model;
- in response to determining that the distribution of the current values corresponds to the given pattern and in case the given pattern is different from the predefined pattern, modify the irradiation plan (199) in accordance with the given pattern, the modified irradiation plan becoming the irradiation plan to be used for the irradiation of the target volume (246).

11. The system of claim 10, wherein each pattern of the patterns (326A-E) is associated with a probability of occurrence, wherein the given pattern is a pattern having the probability of occurrence higher than a predefined threshold.

12. The system of any of claim 10 or 11, wherein the motion model comprises probability of transitions between successive patterns of the patterns (326A-E), the execution of the instructions further causes the system to: determine a future pattern depending on the transition probabilities and the predefined pattern and optionally previous patterns, the modification of the irradiation plan comprising modifying the irradiation plan in accordance with the future pattern.

13. The system of any of the preceding claims 10-12, wherein the motion model comprises an added cluster according to claim 5, wherein in case the given pattern is the added pattern the modifying of the irradiation plan is performed such that the irradiation is stopped.

14. The system of any of the preceding claims, the execution of the machine executable instructions further causing the processor (103) to update the motion model during the irradiation using measured values of the fundamental frequency, breathing amplitude and /or offset of the motion.
